# EUROPEAN PATENT APPLICATION

(11) **EP 3 673 796 A1**
(43) Date of publication of application: **01.07.2020**
(21) Application number: 19220048.3
(22) Date of filing: 30.12.2019
(51) Int. Cl.: A61B 5/00

(54) **USE OF A FLEXIBLE CAPILLARY FOR THE SENSOR DETECTING BIOLOGICALLY ACTIVE MOLECULES**

(30) Priority: 31.12.2018 EP 18215950
(71) Applicant: SDS Optic Spolka Akcyjna, 20-708 Lublin (PL)
(72) Inventor: Staniszewska, Magdalena, 21-030 Motycz (PL); Staniszewski, Marcin, 21-030 Motycz (PL)
(74) Representative: Kondrat, Mariusz

(57) **Abstract**

The subject of invention is the use of a flexible capillary for the sensor detecting biologically active molecules, characterized in that the capillary (1) is a structural element of the fibre-optic probe and the optical fibre is freely placed inside it (2).

## Description

The subject of the invention is the use of a flexible capillary for the sensor detecting biologically active molecules.

The state of the art describes a biosensor-based toxin detection device for milk samples in which the surface of a Fabry-Perot interferometer, which is a capillary-flow glass microplate, has been coated with fragments of antibodies, the so-called Fab' (Chalyan T et al., Asymmetric Mach-Zehnder Interferometer Based Biosensors for Aflatoxin M1 Detection, Biosensors 2016, 6, 1; doi:10.3390/bios6010001).

The US 10/483,586 application (publication no. US20040186359 A) describes a diagnostic apparatus using detection by means of a coated optical fibre; which, however, requires implantation in the body.

The Clinical Chemistry publication of 1991, entitled Evaluation of the Fiber-Optic Antibody-Based Fluoroimmunosensor for DNA Adducts in Human Placenta Samples by T. Vo-Dinh, J. P. Alarie, R. W. Johnson, M. J. Sepaniak and R. M. Santella describes the measurement of DNA adducts by means of fluorescent signal detection by a single optical fibre with antibodies placed in a pocket at the end of the fibre. The device uses laser light to excite and detect the fluorescent signal. The measurement of adducts requires preparation of the sample by hydrolysis in order to achieve the required sensitivity.

Similarly, the apparatus and method of measurement presented in Biosensors and Bioelectronics 2007, Chemiluminescent optical fiber immunosensor for detection of autoantibodies to ovarian and breast cancer-associated antigens by Orly Salama, Sebastien Herrmann, Alina Tziknovsky b, Benjamin Piura, Michael Meirovich, Ilya Trakht, Brent Reed, Leslie I. Lobel, Robert S. Marks requires a marker, e.g. a chemiluminescent one and secondary antibodies for detection. The device is prepared to perform measurements in a solution.

The document Nature Biotechnology 2000, Antibody-based nanoprobe for measurement of a fluorescent analyte in a single cell by Tuan Vo-Dinh, Jean-Pierre Alarie, Brian M. Cullum, and Guy D. Griffin describes another apparatus using an optical fibre coated with antibodies, suitable for use in a single cell. In this device, however, measurement is also based on detection of the fluorescent signal and the device includes a single optical fibre connected to a fluorescence microscope.

The state of the art has already indicated some solutions enabling elimination of the use of markers for detection. The Scientific Reports publication of 2014, entitled Nanoscale Label-free Bioprobes to Detect Intracellular Proteins in Single Living Cells by Wooyoung Hong, Feng Liang, Diane Schaak, Marko Loncar and Qimin Quan describes an apparatus using an antibody-coated optical fibre that does not require fluorescent markers and is based on the surface plasmon resonance technique. The device has been adapted to perform measurements in a single cell and includes an optical fibre terminated with an antibody-coated gold nanotube. When the analyte is connected, the LSPR signal is shifted. Similarly, a Biosensors and Bioelectronics document of 2014, titled An enhanced LSPR fiber-optic nanoprobe for ultrasensitive detection of protein biomarkers, Mollye Sanders, Yongbin Lin, Jianjun Wei, Taylor Bono, Robert G. Lindquist describes an analogous solution, also based on an optical fibre probe and the use of the LSPR signal from an antibody-coated gold nanodisc at the end of the probe. The device is adapted to determine the analyte in a solution. There are no solutions described to carry out measurements directly in the tissue.

Currently known solutions do not enable a simple, minimally invasive determination of analytes directly in tissues, including solid tissues, among others, a solid tumour, whether isolated or in situ directly in the patient, without having to use any markers and without prior tissue preparation. Current diagnostic solutions require sampling, fixation and/or processing and therefore do not reflect the actual condition of the tissue. In addition, devices using the flow of the tested substance through the device (as in the case of microplates) require significant amounts of the sample. The use of a microprobe used for in-situ measurements ensures safety in use and limits the amount of material required for the assay.

A device known from the description of the P.420189 application contains an optical sensor that uses selective interaction with the tested substance of the binding agents immobilised on the surface of the optical fibre which is the interferometer arm, where the optical fibre being the interferometer arm, containing the immobilized binding agent, is fixed inside a guide, preferably a metal one, enabling piercing of the tissue and direct measurement of the substance present in the tissue, so as to detect it and/or determine the concentration. Design of the device, according to the invention, enables it to insert a fibre-based sensor without interfering with the measurement and damaging the sensor itself, directly into the tissue. This device also enables in-situ measurement with an optical fibre-based probe in a safe and minimally invasive way for the patient. The guide also makes it possible to protect the patient from the remaining part of the fibre-optic sensor in the tissue, e.g. should the sensor accidentally crack or break. The optical fibre may be fixed in the guide and thus shielded in part or over its entire length to reduce the effect of any shocks on the measurement result.

The purpose of the invention is to provide a new design of a fibre-optic probe.

The essence of the invention is an use of a flexible capillary for the sensor detecting biologically active molecules, characterized in that the capillary is a structural element of the fibre-optic probe and the optical fibre is freely placed inside it.

Preferably the capillary is made of plastic, metal or glass.

Preferably, the capillary is closed on one side with a light-permeable material being the sensor for collecting the analysed compounds.

Preferably, the light-permeable material is selected from a group comprising a flat disc or a plate made of plastic, a disc or plate made of quartz or a disc or plate made of glass.

Preferably, the capillary has an internal diameter between 1 µm and 3 mm and an external diameter between 2 µm and 5mm.

Preferably, the outer wall of the sensor is located away from the inner wall of the capillary in the range from 0 µm to 1.499 mm.

The invention provides the following advantages:
- protection of the fibre-optic sensor from mechanical damage when placing the fibre-optic sensor in the patient's body;
- mechanical protection of the sensor during transport against breaking and other mechanical damage such as abrasion;
- mechanical protection of the sensor during its use in measuring the specific substance against breaking and other mechanical damage, such as abrasion;
- the use of the resonance cavity effect by closing one end of the capillary with a transparent plate.

The invention is shown in figures, where fig. 1 shows the top view of the capillary; fig. 2 shows the bottom view of the capillary; fig. 3 shows a capillary in an option where the face of the optical fibre is in contact with the inner face of the light-permeable material; fig. 4 shows the capillary in an option where the face of the optical fibre is not in contact with the inner face of the light-permeable material; fig. 5 shows the capillary combined with a rigid fibre-optic sensor.

The invention is presented in embodiments.

### Embodiment 1

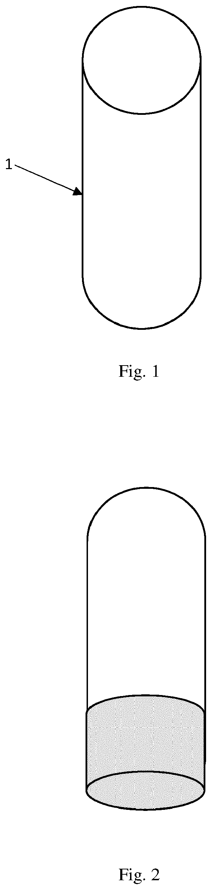

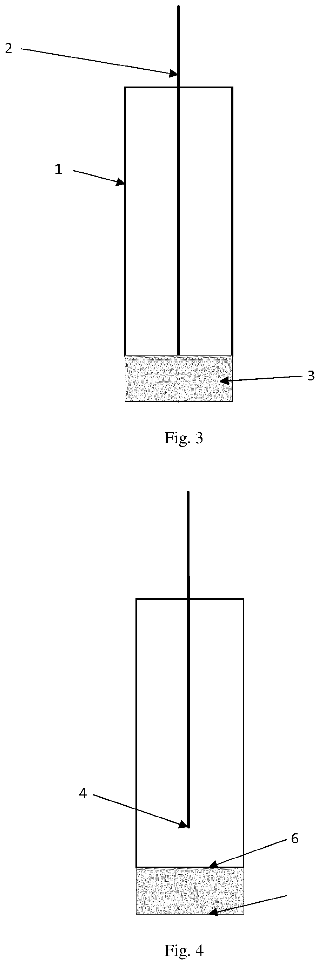

The flexible capillary 1 is shown in fig. 1-2. Whereas Fig. 3-4 shows the use of a flexible capillary for the sensor detecting biologically active molecules, where 1 means capillary, 2 means the optical fibre, 3 means the light-permeable material, 4 means the face of the optical fibre, 5 means the external face of the light-permeable material, 6 means the internal face of the light-permeable material.

Capillary 1 can be made of plastic or metal or glass, which has flexible properties and ensures free insertion of the optical fibre 2 in it. The capillary 1 is a structural element of the fibre-optic probe. One end of the capillary is open and the other is closed by any light-permeable material 3 (such as a flat disc/plate of plastic, quartz, glass) which is a sensor on the surface of which the assayed compounds accumulate.

The flexible capillary 1 has an internal diameter between 1 µm and 3.00 mm and an external diameter between 2 µm and 5.00 mm. Inside the capillary there is any optical fibre 2 which is responsible for the transmission of light from the light source to the sensor and from the sensor to the detector.

In this embodiment, one end of capillary 1 is closed by a disc made of plastic or glass, through which light can pass. The disc can be for example welded to capillary with the GPX-3400 fusion splicer. The diameter of the disc is equal to the outer diameter of capillary or greater in the range from 0.0 mm to 5 mm. The disc acts as for example the sensor's resonance cavity (fig. 2).

The disc on the outside is covered with substances sensitive to the biological or chemical agent being tested, such as the front of the fibre-optic sensor in application PCT_EN2017_050030.

In this embodiment, capillary 1 was produced with a device such as the Microelectrode Puller from World Precision Instruments. The fabrication of the capillary uses processes such as drawing, heating, cooling.

In capillary 1 there is an optical fibre that face 4 at one end is in contact with the inner face 6 of the welded disc (fig. 3) or it is located at a distance of 0.0 mm to 30.0 mm from the inner face of the disc 6 (fig. 4). The other end of the fibre protrudes freely from the other end of the capillary (fig. 3 and fig. 4).

The optical fibre is preferably placed in the capillary 1 in such a way that the external wall of the sensor is distant from the internal wall of capillary 1 in the range from 0 µm to 1.499 mm.

Where the fibre-optic sensor may be any fibre-optic sensor.

### Embodiment 2

Fig. 4 shows the use of a flexible capillary for the sensor detecting biologically active molecules, where 1 means capillary, 2 means the optical fibre, 3 means the light-permeable material, 4 means the face of the optical fibre, 5 means the external face of the light-permeable material, 6 means the internal face of the light-permeable material.

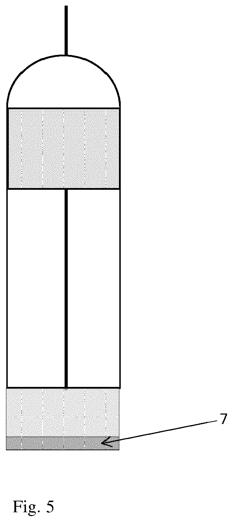

The fibre-optic sensor is placed in a capillary closed on one side 1, whose one-sided closure is a light-permeable material 3, whose outer face 5 is covered with a material sensitive to the analyte 7 (fig. 5) while the inner face 6 of the light-permeable material 3 is separated from the face of the fibre 4 by a distance of 0 µm to 30 mm.

In this embodiment the capillary is a tube open at one end and closed at the other end (fig. 4-5). In this embodiment, the capillary is made of plastic or metal or glass with external dimensions: internal diameter between 1 µm and 3.00 mm and external diameter between 2 µm and 5.00 mm.

Capillary 1 can be made by means of a device such as the Microelectrode Puller from World Precision Instruments. The fabrication of the capillary uses processes such as drawing, heating, cooling.

One end of the capillary 1 is closed by a light-permeable material 3. Whereas the light-permeable material 3 is a material selected from the group including a disc, a quartz plate, a glass plate or a plastic plate. Whereas the light-permeable material 3 is between 1 µm and 3 mm thick.

In this embodiment, one end of capillary 1 is closed by a disc made of plastic or glass, through which light can pass. The disc can be for example welded to capillary 1 with the GPX-3400 fusion splicer. The diameter of the disc is equal to the outer diameter of capillary 1 or greater in the range from 0.0 mm to 5 mm. The disc acts as, for example, the sensor's resonance cavity.

An optical fibre is placed in capillary 1, whose face 4 of one end is in contact with the inner face 6 of the welded disc or is distant from the inner face 6 of the welded disc in the range of 0.0 mm to 30.0 mm. The other end of the fibre is attached to the other end of the capillary and is further free (fig. 4). Preferably, the other end of the optical fibre of the sensor is made rigid against the capillary (1) at the open end of the capillary (1) with an adhesive for medical applications or a weld (fig. 5).

In this embodiment the outer wall of the sensor is located away from the inner wall of capillary in the range from 0 µm to 1.499 mm.

In this embodiment the thickness of the light-permeable disc is between 1 µm and 3.00 mm. The disc can be made e.g. in such a way that a tube made of transparent material e.g. glass, plastic, quartz is welded to the capillary e.g. with a GPX-3400 fusion splicer and then cut to length with a fibre-optic cutter e.g. LDC-400.

The disc is coated on the outside with an analyte-sensitive material 7 e.g. with substances sensitive to the biological or chemical agent being tested, in the same way as the fibre-optic sensor head in application PCT_EN2017_050030.

The disc at the end of the capillary forms a resonance cavity which results in the structure of a reflective interferometer, where the reference beam is the beam reflected from the front of the optical fibre and the measuring beam is the beam reflected from the test substance (analyte) that has deposited on the front of the sensitive disc (covered with a suitable analyte-sensitive material such as the front of a fibre-optic sensor in application PCT_EN2017_050030).

The interference occurs between two beams: the beam reflected from the front face of the fibre at the fibre/disc boundary and the beam reflected from the disc/analyte at the analyte settling point. The use of e.g. polarized fibre limits the influence of external factors, especially temperature changes on the polarization of the beam, i.e. possible fluctuations in the interference contrast.

## Claims

1. An use of a flexible capillary for the sensor detecting biologically active molecules, **characterized in that** the capillary (1) is a structural element of the fibre-optic probe and the optical fibre is freely placed inside it (2).

2. The use according to claim 1, **characterised in that** the capillary is made of plastic, metal or glass.

3. The use according to claim 1 or 2, **characterized in that** the capillary (1) is closed on one side with a light-permeable material (3) being the sensor for collecting the analysed compounds.

4. The use according to claim 3 **characterized in that** the light-permeable material (3) is selected from a group comprising a flat disc or a plate made of plastic, a disc or plate made of quartz or a disc or plate made of glass.

5. The use according to any of the preceding claims 1 to 4, **characterized in that** the capillary has an internal diameter between 1 µm and 3 mm and an external diameter between 2 µm and 5 mm.

6. The use according to any of the previous claims from 1 to 5, **characterized in that** the outer wall of the sensor (2) is located away from the inner wall of the capillary (1) in the range from 0 µm to 1.499 mm.
